# EUROPÄISCHE PATENTSCHRIFT

(11) **EP 3 445 414 B1**
(45) Veröffentlichungstag und Bekanntmachung des Hinweises auf die Patenterteilung: **04.05.2022**
(21) Anmeldenummer: 17718348.0
(22) Anmeldetag: 11.04.2017
(51) Int. Cl.: A61L 27/44, A61L 27/46, A61L 27/54, A61L 27/58

(54) **HYBRIDIMPLANTAT AUS EINEM KOMPOSITMATERIAL**
HYBRID IMPLANT MADE OF A COMPOSITE MATERIAL
IMPLANT HYBRIDE EN MATÉRIAU COMPOSITE

(30) Priorität: 19.04.2016 DE 102016107223
(43) Veröffentlichungstag der Anmeldung: 27.02.2019
(73) Patentinhaber: Karl Leibinger Medizintechnik GmbH & Co. KG, 78570 Mühlheim/Donau (DE)
(72) Erfinder: MILKAU, Daniel, 78532 Tuttlingen (DE); REINAUER, Frank, 78576 Emmingen-Liptingen (DE); WOLFRAM, Tobias, 63303 Dreieich (DE)
(74) Vertreter: Winter, Brandl - Partnerschaft mbB
(86) Internationale Anmeldenummer: PCT/EP2017/058685
(87) Internationale Veröffentlichungsnummer: WO 2017/182333

(56) Entgegenhaltungen:
- EP-A1- 2 730 298
- WO-A1-2017/068492
- WO-A2-2008/106625
- WO-A2-2014/123978
- DE-A1-102004 035 182
- DE-T2- 69 332 346
- US-A1- 2015 071 983
- US-B1- 6 432 437
- BASEL SHARAF ET AL: "Three-Dimensionally Printed Polycaprolactone and ?-Tricalcium Phosphate Scaffolds for Bone Tissue Engineering: An In Vitro Study", JOURNAL OF ORAL AND MAXILLOFACIAL SURGERY., Bd. 70, Nr. 3, 1. März 2012 (2012-03-01), Seiten 647-656, XP55387670, US ISSN: 0278-2391, DOI: 10.1016/j.joms.2011.07.029
- ANNA MORAWSKA-CHOCHÓL ET AL: "Gentamicin release from biodegradable poly-l-lactide based composites for novel intramedullary nails", MATERIALS SCIENCE AND ENGINEERING C., Bd. 45, 1. Dezember 2014 (2014-12-01), Seiten 15-20, XP55387758, CH ISSN: 0928-4931, DOI: 10.1016/j.msec.2014.08.059
- SCHILLER C ET AL: "Geometrically structured implants for cranial reconstruction made of biodegradable polyesters and calcium phosphate/calcium carbonate", BIOMATERI, ELSEVIER SCIENCE PUBLISHERS BV., BARKING, GB, Bd. 25, Nr. 7-8, 1. März 2004 (2004-03-01) , Seiten 1239-1247, XP004475068, ISSN: 0142-9612, DOI: 10.1016/J.BIOMATERIALS.2003.08.047

## Beschreibung

Die vorliegende Erfindung betrifft ein Hybridimplantat aus einem Kompositmaterial.

Als benachbarter Stand der Technik ist beispielsweise aus der DE 102008040782 A1 ein Verfahren zur Herstellung von medizinischen Hybridimplantaten bekannt, mit wenigstens einer ersten Komponente aus Metall und/oder Keramik und/oder Kunststoff und wenigstens einer zweiten Komponente aus Kunststoff, dadurch gekennzeichnet, dass vor einem Verbinden der Komponenten auf der oder den Oberfläche(n) der ersten Komponente zumindest teilweise mittels elektromagnetischer Strahlung eine Oberflächenstruktur erzeugt wird, die eine von einer Nanostruktur überlagerte Mikrostruktur aufweist, und anschließend die zweite Komponente aufgebracht wird.

Gattungsbildender Stand der Technik eines Hybridmoduls, dessen keramisch-anorganische Komponente Calciumcarbonat enthält, ist aus der DE 693 32 346 T2 oder aus der WO 2014/123 978 A2 bekannt.

Zudem sind aus der EP 2 730 298 A1, der WO 2008/108 106 625 A2 und der WO 2017/068 492 A1 Hybridimplantate aus einem Kompositmaterial bekannt. Weiterhin betreffen die DE 10 2004 035 182 A1, der US 2015/071983 A1 oder der US 6 432 437 B1 Hybridimplantate mit einem Polymer und Calciumcarbonatpartikeln.

In der modernen Implantattechnologie steht die Konzeption intelligenter Implantate, welche neben ihrer mechanischen bzw. Stützfunktion aktiv mit dem sie umgebenden Gewebe in Wechselwirkung treten, zunehmend im Fokus der Entwicklung.

Bei der Entwicklung derartiger Implantate wird vor Allem auf zunehmende Kenntnisse der Bedingungen, wie z.B. der Botenstoffkaskaden, in traumatisiertem bzw. heilendem Gewebe zurückgegriffen, um die Implantate derart zu konzipieren, dass sie lokal um das Implantat herum ein für die Gewebeheilung möglichst zuträgliches Nischenklima erzeugen. Ein solches Nischenklima wird durch eine biologische Nische bedingt. Unter einer biologischen Nische werden hierbei die durch das lokal begrenzte Zusammenwirken von biologischen, chemischen, physikalischen und strukturellen Faktoren erzeugten Bedingungen bzw. das erzeugte Milieu verstanden. Also ist darunter eine zelluläre Nische auf Mikroniveau zu verstehen.

Da das Implantat meistens vollständig implantiert werden soll, d.h. ohne dass zu einem späteren Zeitpunkt eine Einwirkungsmöglichkeit auf das Implantat durch einen Operateur besteht, ist es bei der Entwicklung derartiger Implantate besonders wichtig, dass die Eigenschaften des Implantats im Vorhinein verlässlich und reproduzierbar zur Erzeugung eines definierten Nischenklimas eingestellt werden können.

Ein aus dem Stand der Technik bekanntes Implantat, welches durch die Abgabe von Botenstoffen bzw. Faktoren ein für die Heilung besonders zuträgliches Nischenklima erzeugen soll, ist beispielsweise ein Implantat in Form eines mit einem bestimmten Botenstoff bzw. Faktor getränkten Schwämmchens aus Kollagen. Ist ein derartiges Implantat in einen Körper eingebracht, diffundieren die in dem Schwämmchen enthaltenen Faktoren aus dem Schwämmchen in das umliegende Gewebe und können somit die Heilung des umliegenden Gewebes fördern.

Ein derartiges Implantat hat jedoch die folgenden Nachteile:
Soll die Diffusion des Botenstoffes oder Faktors aus dem Schwämmchen für einen längeren Zeitraum gewährleistet werden, ist es zunächst nötig, das Schwämmchen mit einer Botenstofflösung zu tränken, deren Konzentration weit über der physiologischen Konzentration dieses Botenstoffes liegt. Dies hat negative Auswirkungen auf die körpereigenen "Signalling-Kaskaden", wirkt also zeitlich und räumlich undefiniert und kann nicht gezielt abgeschaltet werden. Zudem erfolgt die Diffusion des Botenstoffs in das Gewebe unkontrolliert und daher nicht mit einer einheitlichen Rate bzw. Konzentration über die gesamte Oberfläche des Implantats, hat dann also negative Auswirkungen auf die Effizienz und Wirkweise. Daraus können gefährliche Gewebereaktionen entstehen, die eher pathologischen Überexpressionen von Wachstums- oder Signalmolekülen entsprechen als den körpereigenen regenerativen Gewebereaktionen.

Ein weiterer Nachteil liegt momentan darin, dass Kollagen verwendet wird, welches selbst im Körper bereits vorkommt und als Botenstoff agieren kann. Somit können unerwünschte Nebenwirkungen durch die Funktion des Kollagens in "Signalling-Kaskaden" entstehen. Dieser Effekt kann insbesondere in Geweben mit relativ geringem intrinsischem Kollagenanteil auftreten. Ergo können Prozesse angeregt werden, die eigentlich ungewünscht sind.

Ein zusätzlicher Nachteil besteht darin, dass zur Fertigung kollagenbasierter Implantate meist auf Kollagen tierischen Ursprungs, beispielsweise auf equines Kollagen, zurückgegriffen wird. Derartiges Kollagen unterliegt als Naturprodukt jedoch höheren Qualitätsschwankungen als voll-synthetische Materialien, was zu Qualitätsschwankungen bzw. zu einer weniger verlässlichen Reproduktion bzw. Definition der Eigenschaften eines kollagenbasierten Implantats führt.

Weiterhin sind aus dem Stand der Technik Implantate bekannt, deren Zusammensetzung derart ausgewählt ist, dass das Implantat im Körper möglichst neutral bzw. passiv wirkt. Ein Beispiel hierfür ist die Auswahl von Bestandteilen eines Implantats zur Erlangung eines möglichst neutralen pH- Werts beim Abbau des Implantats im Körper.

Derartige Implantate wirken jedoch nicht positiv auf die Schaffung einer für die Gewebeheilung zuträglichen biologischen Nische hin, vielmehr soll bei derartigen Implantaten vor allem eine negative Wirkung des Implantats auf die Wundheilung vermieden bzw. neutralisiert werden, sodass sich das Implantat im Körper biologisch passiv verhält.

Der vorliegenden Erfindung liegt somit die Aufgabe zugrunde, ein Implantat bereitzustellen, welches die aus dem Stand der Technik bekannten Nachteile vermindert oder sogar beseitigt, insbesondere soll durch seine präzise und reproduzierbar einstellbaren Eigenschaften nach der Implantation aktiv eine der Gewebeheilung zuträgliche biologische Nische generiert werden. Dabei soll bewusst auf biologisch aktive, protein-basierte Signalmoleküle verzichtet werden, da deren biochemische Aktivität in den Heilungs- und Wirkungsprozessen nicht ausreichend kontrolliert werden kann und deren Stabilität in den spezifischen räumlichen Strukturen nicht definiert bzw. vorhersehbar oder kontrolliert ist.

Vorteilhafterweise werden nur nicht- proteinbasierte Signalmoleküle bzw. Botenstoffe und / oder nicht-proteinogene Signalmoleküle bzw. Botenstoffe im Rahmen eines erfindungsgemäßen Implantats verwendet. Somit ist das gesamte Hybridimplantat bzw. Poly-Hybridimplantat vorzugsweise im Wesentlichen frei von Proteinen, proteinbasierten Stoffen und proteinogenen Stoffen. Besonders bevorzugt ist das gesamte Hybridimplantat bzw. Poly-Hybridimplantat vollständig frei von Proteinen, proteinbasierten Stoffen und proteinogenen Stoffen.

Diese Aufgabe wird durch das Implantat mit den Merkmalen des Anspruchs 1 gelöst, insbesondere durch ein Hybridimplantat aus Kompositmaterial, das eine Polymermatrix und eine keramisch-anorganische Komponente aufweist, wobei die Polymermatrix wenigstens einen Bestandteil aufweist, welcher ausgewählt ist aus der Gruppe PDLLA, PLGA, PCL, HDPE, PE, UHMWPE, PEAK, PEEK, PP, PUR, und die keramisch-anorganische Komponente wenigstens einen Bestandteil aufweist, welcher ausgewählt ist aus der Gruppe HAP, α-TCP, β-TCP und CaCO₃. Erfindungsgemäß enthält die keramisch-anorganische Komponente CaCO₃, wobei das CaCO₃ zwischen ca. 15 % und ca. 65 %Gewichtsanteil zu der Gesamtmasse des Hybridimplantats beiträgt. Ferner weist das Kompositmaterial erfindungsgemäß zusätzlich metallische Bestandteile auf, wobei die metallischen Bestandteile zwischen ca. 5 % und ca. 15 % Gewichtsanteil zu der Gesamtmasse des Hybridimplantats beitragen.

Durch die Auswahl seiner Bestandteile und seine geometrische Ausgestaltung wird eine zur Förderung der Gewebeheilung vorteilhafte biologische Aktivität bzw. eine vorteilhafte biologische Nische erzeugt.

Der Kern der Erfindung liegt hierbei darin, dass das Implantat als "Poly"-Hybridimplantat aus einem Kompositmaterial ausgebildet ist und vollständig aus künstlich synthetisierten Materialien besteht. Das Kompositmaterial weist eine Polymermatrix und mindestens eine keramisch-anorganische oder mindestens eine anorganische Komponente oder verschiedene Kombinationen dieser Materialien auf. Diese Komponenten werden in definierten Verhältnissen miteinander in das fertige Implantat eingebracht, um die Knochenregeneration optimal zu steuern.

Die Polymermatrix kann je nach Anwendung aus vollständig biodegradierbaren Polymeren, teilweise biodegradierbaren Polymeren oder aber nicht biodegradierbaren Polymeren bestehen. Hierbei sind Misch- und Reinpolymere möglich.

Als besonders vorteilhaft haben sich die folgenden Polymere herausgestellt: Poly-DL-Lactid (Poly-DL-Lactic Acid, PDLLA); Polylactid-co-Glycolid (Poly-Lactic-co-Glycolic-Acid, PLGA), Polycaprolacton (PCL), High- density poly-etylene (HDPE), Polyethylen (Polyethylene, PE), Ultra high molecular weight - Polytethylene (UHMWPE), Polyaryletherketon (Polyaryletherketone, PEAK), Polyetheretherketon (Polyetherketone, PEEK), Polypropylen (PP), Polyurethan (PUR).

Die keramisch-anorganische Komponente weist wenigstens einen Calcium-basierten, beispielsweise Calciumphosphat- basierten (CaP-basierten), Bestandteil auf, welcher vorzugsweise ausgewählt ist aus der Gruppe Hydroxylapatit (HAP), α-Tricalciumphosphat (a-TCP), β-Tricalciumphosphat (β-TCP) und Calciumcarbonat (CaCO₃). Erfindungsgemäß enthält die keramisch-anorganische Komponente Calciumcarbonat, das zwischen ca. 15 % und ca. 65 % Gewichtsanteil zu der Gesamtmasse des Hybridimplantats beiträgt.

Durch die Verwendung sowohl einer organischen Komponente, der Polymermatrix, und einer keramisch- anorganischen oder anorganischen Komponente, lassen sich die Eigenschaften des Hybridimplantats verbessert einstellen.

So kann beispielsweise eine nicht biodegradierbare Polymermatrix zur dauerhaften Wahrnehmung einer Stützfunktion vorgesehen sein, während die biodegradierbare keramische Komponente beispielsweise durch Freisetzung von Calcium-Ionen positiv eine Gewebeinduktion beispielsweise durch eine Aktivierung von Chondrozyten und / oder Osteoblasten bewirkt.

In einem derartigen Fall liegen die induktiven Faktoren, in diesem Fall Ionen, primär nicht in Lösung vor, sodass auf die im Stand der Technik verwendeten hochkonzentrierten Lösungen von Molekülen, wie z.B. Wachstumsfaktoren, verzichtet werden kann. Vielmehr werden die Ionen durch die Degradation des Implantats an der gesamten Oberfläche des Implantats gleichmäßig freigesetzt, in Lösung gebracht und erhöhen somit nicht die Konzentration der natürlich vorkommenden Komponenten über den Zeitraum der Wirkung der Implantate Dieser Prozess kann optimiert eingestellt werden durch die Verfahren zur Herstellung der Hybrid- oder "Poly"-Hybridimplantate.

Durch die Anpassung der Zusammensetzung der Bestandteile des Implantats und der Anordnung bzw. der Verarbeitung dieser Bestandteile (z.B. Kompaktierung, Vorliegen bestimmter Bestandteile in definierten Geometrien oder Gradienten etc.) lassen sich somit die Abgabeparameter der Faktoren, wie beispielsweise die Abgaberate oder der Zeitverlauf der Abgabe, genau einstellen.

Vorteilhafte Weiterbildungen eines erfindungsgemäßen Implantats sind Gegenstand der Unteransprüche und werden nachfolgend näher beschrieben.

Als besonders vorteilhaft hat es sich erwiesen, wenn das CaCO₃ bevorzugt ca.15 % - ca. 35 %, insbesondere ca. 15 % - ca. 25 % oder ca. 25 % - ca. 35 %, oder ca. 50 % - ca. 65% Gewichtsanteil zu der Gesamtmasse des Hybridimplantats beiträgt.

CaCO₃ dient hierbei als Abbaubeschleuniger beim Abbau bestimmter biodegradierbarer Polymere und wirkt zudem als osteokonduktive Substanz, die ein festes Einwachsen neugebildeten Gewebes in ein Implantat fördert. Dies ist insbesondere wünschenswert, wenn die Polymermatrix des Hybridimplantats zumindest teilweise aus nicht- biodegradierbaren Polymeren besteht und somit eine dauerhafte Gerüststruktur für das einwachsende Gewebe bildet. Dadurch entsteht eine neue und innovative Möglichkeit, die Implantat-Gewebereaktion während der Gewebeneubildung quantitativ und räumlich zu steuern.

Das CaCO₃ kann als im Wesentlichen sphärische Partikel vorliegen, wobei die sphärischen Partikel mindestens eine Gruppe von sphärischen Partikeln mit einem Durchmesser ausgewählt aus dem Bereich von ca. 10 µm - ca. 200 µm, insbesondere aus den Bereichen von ca.10 µm - ca.15 µm; ca. 20 µm - ca. 25 µm; ca. 30 µm - ca. 45 µm; ca. 100 µm - ca. 200 µm umfassen. In anderen Worten können alle CaCO₃-Partikel denselben Durchmesser aufweisen, oder das CaCO₃ kann in Form von Partikeln mit unterschiedlich großen Durchmessern vorliegen. Die jeweils ausgewählten Größen an Durchmesser sind ergo immer bewusst im Vorhinein ausgewählt oder eingestellt.

Zudem können die CaCO₃- Partikel eine spezifische Oberflächenmodifaktion, beispielsweise zur Erlangung einer lipophilen oder hydrophilen Oberfläche, und / oder eine definierte Topographie aufweisen. Auch andere Partikel, welche keine CaCO₃-Partikel sind, oder sonstige Bestandteile eines erfindungsgemäßen Implantats inklusive der Polymermatrix können eine derartige Oberflächenmodifikation und / oder Topgraphie aufweisen.

Zusätzlich können die metallischen Bestandteilen ausgewählt sein aus der Gruppe Magnesium, Eisen, Zink und Strontium. Natürlich ist eine Mischung auch möglich. Diese metallischen Bestandteile bieten weitere Möglichkeiten, die Eigenschaften des Implantats für die Erzeugung einer bestimmten biologischen Nische einzustellen.

Erfindungsgemäß tragen die metallischen Bestandteile zwischen ca. 5% bis ca. 15 % Gewichtsanteil zur Gesamtmasse des Hybridimplantates bei.

Beispielsweise können die metallischen Bestandteile als Partikel, Fasern oder Späne vorliegen, deren Eigenschaften wiederum bei zumindest teilweise biodegradierbaren Implantaten den Abbau des Implantats beeinflussen, sowie auch auf das umliegende Gewebe einwirken können.

Derartige metallische Bestandteile werden meist in Form von Partikeln eingesetzt. Die Formgebung der Partikel kann hierbei unregelmäßig, im Wesentlichen sphärisch oder im Wesentlichen faserförmig oder im Wesentlichen verdrillt oder im Wesentlichen helikal strukturiert sein.

Weiterhin kann das vollständig oder nicht-vollständig resorbierbare Implantat eine spezifische Gerüst- bzw. "Scaffold"-Struktur aufweisen. Derartige Gerüststrukturen fördern das Einwachsen von Gewebe um bzw. in das Implantat.

Eine derartige Gerüststruktur kann vorteilhafterweise Hohlstrukturen aufweisen. Diese Hohlstrukturen sind bevorzugt interkonnektierend und besitzen Durchmesser von ca. 100 µm - ca. 800 µm. Die Ausgestaltung der Hohlstrukturen kann anwendungsspezifisch angepasst werden. Durch die Verwendung von bestimmten Produktionstechnologien, wie z.B. bestimmten 3D-Printing Verfahren, können Hohlstrukturen mit einer technisch genau definierten und einstellbaren Größe und Verteilung bzw. Anordnung gebildet werden. Dazu kann zunächst eine Komponente genutzt werden (entweder Polymer, oder keramische oder anorganische Komponente), anschließend wird die zweite und eventuell weitere Komponenten um die primäre Stütz- und Hohlstruktur herum gebaut.

Eine derartige Gerüst- oder "Scaffold"-Struktur kann ausschließlich durch die Polymermatrix gebildet sein, es kann aber auch ein Verbund aus Polymermatrix und anorganisch-keramischer Komponente als Gerüst- oder "Scaffold"-Struktur fungieren.

Durch die anwendungsspezifische Ausführung der Implantate, insbesondere deren struktureller Ausgestaltung und chemischer Zusammensetzung wird eine optimale Nutzung von räumlichen Nischen zum endogenen Aufbau von Körpergewebe ermöglicht.

Um zusätzlich aktiv zur Erzeugung einer derartigen biologischen Nische beizutragen, kann das Hybridimplantat weiterhin gewebeinduktive und / oder gewebekonduktive Faktoren, insbesondere nicht-protein-basierte chemische Botenstoffe und / oder Ionen, oder auch antibiotische Wirkstoffe enthalten.

Ein weiterer Vorteil des erfindungsgemäßen Hybridimplantats besteht darin, dass das Implantat mit einem sinterlosen 3D- additiven Verfahren bei einer relativ geringen Temperatur, d.h. zwischen Raumtemperatur (etwa 25°C) und ca. 250 °C gefertigt werden kann.

Herkömmliche Implantate werden meist nach der Herstellung einem Sinterschritt unterzogen, um die gewünschten mechanischen Eigenschaften des Implantats zu erreichen. Durch die Eigenschaften der verwendeten Ausgangsmaterialen entfällt bei der Herstellung des erfindungsgemäßen Implantats jedoch die Notwendigkeit des Sinterns, wodurch das Herstellungsverfahren verkürzt und vereinfacht wird.

Vorteilhafterweise wird ein erfindungsgemäßes Implantat in einem additiven dreidimensionalen (3D) - Verfahren hergestellt. Als mögliches 3D - Herstellungsverfahren bieten sich insbesondere die im Folgenden beispielhaft aufgeführten Verfahren an:
3D - Drucken mit Pulver (3DP), Selektives Lasersintern (SLS), Selective Heat Sintering (SHS), Selektives Laserschmelzen (SLM, Selective Laser Melting), Elektronenstrahlschmelzen (EBM - Electron Beam Melting), Fused Deposition Modeling (FDM, Schmelzschichtung), Fused Filament Fabrication (FFF), Stick Deposition Molding (SDM), Multi-Jet Modeling (MJM), Stereolithografie (STL oder auch SLA), Scan-LED-Verfahren (SLT) als Weiterentwicklung der klassischen Stereolithografie, Film Transfer Imaging (FTI), Digital Light Processing (DLP), PolyJet Laminated Object Modeling (LOM) oder Folienlaminier-3D-Druck, Selective Deposition Lamination (SDL).

Bestimmte Prozesse sind hierbei unter Schutzgas, z.B. Argon oder Stickstoff, durchzuführen.

Prinzipiell kann ein erfindungsgemäßes Implantat jedoch auch in einem subtraktiven Verfahren gefertigt werden, bei dem zunächst aus den Ausgangsmaterialien in den gewünschten Mengen bzw. mit der gewünschten Stoffzusammensetzung beispielsweise durch Pressung eine Ausgangsplatte bzw. ein Ausgangsblock erzeugt wird, der dann z.B. spanend weiterverarbeitet werden kann.

Dadurch, dass das Implantatmaterial sowohl additiv als auch subtraktiv bearbeitet werden kann, und somit auch das Implantat sowohl mit einem additiven als auch mit einem subtraktiven Verfahren erzeugt werden kann, erhöht sich die Vielseitigkeit der Anwendungsmöglichkeiten des erfindungsgemäßen Implantats.

Eine vorteilhafte Ausführungsform eines erfindungsgemäßen Implantats ist ein vollständig biodegradierbares Implantat, welches CaCO₃ enthält. Das CaCO₃ trägt ca. ca. 15 % - ca. 25% zu der Gesamtmasse des Hybridimplantats bei. Die Polymermatrix weist PDLLA, PCL oder PLGA oder einer Mischung aus diesen Polymeren auf.

Zudem enthält das Hybridimplantat Mg-Partikel oder Legierungen, wie z.B. die Magnesiumlegierung WE43 oder MgCa, mit einem Gewichtsanteil zur Gesamtmasse von ca. 5%- ca.15 %. Das CaCO₃ liegt in Partikelform vor. CaCO₃-Partikel weisen einen Durchmesser von ca. 10 µm - ca.15 µm auf und sind annährend sphärisch.

Das Implantat besitzt zudem eine Gerüststruktur mit Hohlräumen mit einem Durchmesser von ca. 300µm - ca. 450µm.

Es sei angemerkt, dass die Erfindung also ein Implantat betrifft, das vollständig oder teilweise biodegradierbar ist.

## Patentansprüche

1. Hybridimplantat aus einem Kompositmaterial, welches eine Polymermatrix und eine keramisch-anorganische Komponente aufweist, wobei die Polymermatrix wenigstens einen Bestandteil aufweist, welcher ausgewählt ist aus der Gruppe PDLLA, PLGA, PCL, HDPE, PE, UHMWPE, PEAK, PEEK, PP, PUR und
die keramisch-anorganische Komponente wenigstens einen Bestandteil aufweist, welcher ausgewählt ist aus der Gruppe HAP, α-TCP, β-TCP und CaCO₃, **dadurch gekennzeichnet, dass** die keramisch-anorganische Komponente CaCO₃ enthält und das CaCO₃ zwischen ca. 15 % und ca. 65 % Gewichtsanteil zu der Gesamtmasse des Hybridimplantats beiträgt, und das Kompositmaterial zusätzlich metallische Bestandteile aufweist und die metallischen Bestandteile zwischen ca. 5 % und ca. 15 % Gewichtsanteil zu der Gesamtmasse des Hybridimplantats beitragen.

2. Hybridimplantat nach Anspruch 1, **dadurch gekennzeichnet, dass** das CaCO₃ zwischen ca. 15 % - ca. 25 % oder ca. 25 % - ca. 35 % oder ca. 50 % - ca. 65% Gewichtsanteil zu der Gesamtmasse des Hybridimplantats beiträgt.

3. Hybridimplantat nach Anspruch 1 oder 2, **dadurch gekennzeichnet, dass** das CaCO₃ als im Wesentlichen sphärische Partikel vorliegt, wobei die sphärischen Partikel mindestens eine Gruppe von sphärischen Partikeln mit einem Durchmesser ausgewählt aus ca. 10 µm - ca. 15 µm, ca. 20 µm - ca. 25 µm, ca. 30 µm - ca. 45 µm, ca. 100 µm - ca. 200 µm umfassen.

4. Hybridimplantat nach Anspruch 3, **dadurch gekennzeichnet, dass** die sphärischen Partikel an ihrer Oberfläche eine definierte Topographie und / oder eine vorzugsweise hydrophile oder lipophile Oberflächenmodifikation aufweisen.

5. Hybridimplantat nach einem der vorhergehenden Ansprüche, **dadurch gekennzeichnet, dass** die metallischen Bestandteile ausgewählt sind aus der Gruppe Magnesium, Eisen, Zink und Strontium.

6. Hybridimplantat nach Anspruch 5, **dadurch gekennzeichnet, dass** die metallischen Bestandteile eine unregelmäßige Formgebung aufweisen oder im Wesentlichen sphärisch, faserförmig, verdrillt oder helikal ausgestaltet sind.

7. Hybridimplantat nach einem der vorhergehenden Ansprüche, **gekennzeichnet durch** interkonnektierende Hohlstrukturen als Gerüststruktur zur Gewebekonduktion mit einem Durchmesser von ca. 100 µm - ca. 800 µm, bevorzugt von ca. 300 µm - ca. 450 µm.

8. Hybridimplantat nach einem der vorhergehenden Ansprüche, **dadurch gekennzeichnet, dass** das Hybridimplantat zusätzlich gewebeinduktive und / oder gewebekonduktive Faktoren, insbesondere nicht-proteinogene chemische Botenstoffe und / oder Ionen, und / oder sonstige Wirkstoffe enthält.

9. Fertigung eines Hybridimplantats nach einem der vorhergehenden Ansprüche, **dadurch gekennzeichnet, dass** ein sinterloses 3D - additives Verfahren bei einer Temperatur zwischen Raumtemperatur und ca. 250 °C eingesetzt wird.

10. Fertigung eines Hybridimplantats nach einem der Ansprüche 1 bis 8, **dadurch gekennzeichnet, dass** ein subtraktives Abtragen eines Ausgangsmaterials eingesetzt wird.

## Claims

1. A hybrid implant made of a composite material having a polymer matrix and a ceramic-inorganic component, wherein the polymer matrix has at least one component selected from the group of PDLLA, PLGA, PCL, HDPE, PE, UHMWPE, PEAK, PEEK, PP, PUR, and
the ceramic-inorganic component contains at least one constituent selected from the group consisting of HAP, α-TCP, β-TCP and CaCO₃, **characterized in that** the ceramic-inorganic component contains CaCO₃ and the CaCO₃ contributes between about 15% and about 65% percentage by weight to the total mass of the hybrid implant, and the composite material additionally contains metallic constituents and the metallic constituents contribute between about 5% and about 15% percentage by weight to the total mass of the hybrid implant.

2. The hybrid implant according to claim 1, **characterized in that** the CaCO₃ contributes between about 15% - about 25%, or about 25% - about 35%, or about 50% - about 65% percentage by weight to the total mass of the hybrid implant.

3. The hybrid implant according to claim 1 or 2, **characterized in that** the CaCO₃ is present as essentially spherical particles, wherein the spherical particles comprise at least one group of spherical particles having a diameter selected from about 10 µm - about 15 µm, about 20 µm - about 25 µm, about 30 µm - about 45 µm, or about 100 µm - about 200 µm.

4. The hybrid implant according to claim 3, **characterized in that** the spherical particles on their surface show a defined topography and/or a preferably lipophilic surface modification.

5. The hybrid implant according to one of the preceding claims, **characterized in that** the metallic components are selected from the group of magnesium, iron, zinc, and strontium.

6. The hybrid implant according to claim 5, **characterized in that** the metallic components show an irregular shaping or are configured to be substantially spherical, fibrous, twisted or helical.

7. The hybrid implant according to one of the preceding claims, **characterized by** interconnecting hollow structures as a scaffold structure for tissue conduction with a diameter of about 100 µm - about 800 µm, preferably of about 300 µm - about 450 µm.

8. The hybrid implant according to claim 1, **characterized in that** the hybrid implant additionally contains tissue-inductive and/or tissue-conductive factors, in particular non-proteinogenic chemical messengers and/or ions, and/or other active agents.

9. A manufacture of a hybrid implant according to one of the preceding claims, **characterized in that** a sinter-less 3D additive method is employed at a temperature between room temperature and about 250°C.

10. The manufacture of the hybrid implant according to one of claims 1 to 8, **characterized in that** subtractive removal of a starting material is used.

## Revendications

1. Implant hybride en matériau composite, qui présente une matrice polymère et un composant inorganique céramique, dans lequel la matrice polymère présente au moins un constituant qui est sélectionné dans le groupe constitué de PDLLA, PLGA, PCL, HDPE, PE, UHMWPE, PEAK, PEEK, PP, PUR et
le composant inorganique céramique présente au moins un constituant qui est sélectionné dans le groupe constitué de HAP, α-TCP, ß-TCP et CaCO₃, **caractérisé en ce que** le composant inorganique céramique contient du CaCO₃ et le CaCO₃ contribue à hauteur d'un rapport en poids entre environ 15 % et environ 65 % à la masse totale de l'implant hybride, et le matériau composite présente en outre des constituants métalliques et les constituants métalliques contribuent à hauteur d'un rapport en poids entre environ 5 % et environ 15 % à la masse totale de l'implant hybride.

2. Implant hybride selon la revendication 1, **caractérisé en ce que** le CaCO₃ contribue à hauteur d'un rapport en poids entre environ 15 °% à environ 25 % ou environ 25 % à environ 35 % ou environ 50 % à environ 65 % à la masse totale de l'implant hybride.

3. Implant hybride selon la revendication 1 ou 2, **caractérisé en ce que** le CaCO₃ est présent en tant que particules pour l'essentiel sphériques, dans lequel les particules sphériques comprennent au moins un groupe de particules sphériques avec un diamètre sélectionné parmi environ 10 µm à 15 µm, environ 20 µm à environ 25 µm, environ 30 µm à environ 45 µm, environ 100 µm à environ 200 µm.

4. Implant hybride selon la revendication 3, **caractérisé en ce que** les particules sphériques présentent au niveau de leur surface une topographie définie et/ou une modification de surface de préférence hydrophile ou lipophile.

5. Implant hybride selon l'une quelconque des revendications précédentes, **caractérisé en ce que** les constituants métalliques sont sélectionnés parmi le groupe constitué de magnésium, de fer, de zinc et de strontium.

6. Implant hybride selon la revendication 5, **caractérisé en ce que** les constituants métalliques présentent une mise en forme irrégulière ou sont pour l'essentiel torsadés de manière sphérique, en forme de fibres ou configurés de manière hélicoïdale.

7. Implant hybride selon l'une quelconque des revendications précédentes, **caractérisé par** des structures creuses s'interconnectant en tant que structure de cadre pour une conduction tissulaire avec un diamètre d'environ 100 µm à environ 800 µm, de préférence d'environ 300 µm à environ 450 µm.

8. Implant hybride selon l'une quelconque des revendications précédentes, **caractérisé en ce que** l'implant hybride contient en outre des facteurs inducteurs de tissu et/ou conducteurs de tissu, en particulier des substances messagères chimiques non protéinogènes et/ou des ions, et/ou divers ingrédients actifs.

9. Fabrication d'un implant hybride selon l'une quelconque des revendications précédentes, **caractérisée en ce qu'**un procédé additif 3D sans frittage est mis en œuvre à une température entre la température ambiante et environ 250 °C.

10. Fabrication d'un implant hybride selon l'une quelconque des revendications 1 à 8, **caractérisée en ce qu'**une élimination par soustraction d'un matériau initial est mise en œuvre.
